# EUROPEAN PATENT SPECIFICATION

(11) **EP 0 810 027 B1**
(45) Date of publication and mention of the grant of the patent: **05.06.2002**
(21) Application number: 96901994.2
(22) Date of filing: 09.02.1996
(51) Int. Cl.: B01J 20/22, C07K 14/525, A61M 1/36, B01J 20/32

(54) **ADSORBENT FOR TUMOR NECROSIS FACTOR ALPHA, METHOD OF REMOVAL BY ADSORPTION, AND ADSORPTION DEVICE USING SAID ADSORBENT**
ADSORBENS FÜR TUMORNEKROSEFAKTOR ALPHA, METHODE ZUR ENTFERNUNG DURCH ADSORPTION UND ADSORPTIONSVORRICHTUNG UNTER VERWENDUNG DES ADSORBENS
ADSORBANT DU FACTEUR ALPHA DE NECROSE TUMORALE, PROCEDE PERMETTANT DE L'ELIMINER PAR ADSORPTION ET DISPOSITIF D'ADSORPTION UTILISANT LEDIT ADSORBANT

(30) Priority: 16.02.1995 JP 2812995
(43) Date of publication of application: 03.12.1997
(73) Proprietor: KANEGAFUCHI KAGAKU KOGYO KABUSHIKI KAISHA, Kita-ku Osaka-shi Osaka-fu 530 (JP)
(72) Inventor: HIRAI, Fumiyasu, Hyogo 661 (JP); TANI, Nobutaka, Osaka 545 (JP); ASAHI, Takashi, Hyogo 655 (JP)
(74) Representative: VOSSIUS & PARTNER
(86) International application number: JP9600306
(87) International publication number: WO9625228

(56) References cited:
- EP-A- 0 306 617
- EP-A- 0 321 703
- EP-A- 0 592 989
- JP-A- 1 068 272
- JP-A- 1 119 264
- JP-A- 1 171 638
- JP-A- 1 181 875
- JP-A- 6 211 900

## Description

The present invention relates to an adsorbent for tumor necrosis factor-α (hereinafter referred to as "TNF-α"), a process for adsorbing and removing TNF-α by using the adsorbent, and an adsorber for TNF-α by using the adsorbent.

Septicemia means a condition wherein a generalized inflammatory reaction occurs because of an infection somewhere in the body. When this inflammatory symptom progresses, a shock symptom occurs (septic shock) and an organopathy occurs (organ failure), and further, a patient comes to have severe conditions such as a multiple organ failure.

Typical substances which cause an inflammation from an infection in this way are various kinds of cytokines and activated complements which are produced by the presence of an endotoxin in the surface of a Gram-negative bacterium, and TNF-α is regarded as the most important as the blood level of TNF-α among such cytokines increases at first and TNF-α promotes the production of other cytokines.

Septicemia can be caused by a Gram-positive bacterium as well as by a Gram-negative bacterium. In this case, it is considered that septicemia is caused by a cytokine such as TNF-α, or the like which is produced by various stimulations owing to local infections even if the endotoxin is not present. Also, in septicemia, when the blood level of TNF-α is kept high, prognosis thereof becomes bad, and the level of TNF-α reflects well a degree of severe condition ("Shucyu Ciryo" 6(2), 115-123 (1994)).

As a conventional therapy for such septicemia, there is a therapy wherein an antibiotic is administrated as an infection measure or a therapy wherein γ-globulin is administrated in order to activate resistance to the infection. However, the mortality rate is still high. So, it is desired from a medical standpoint to remove from a body fluid TNF-α which is regarded as the most important among cytokines which cause inflammation.

It is known that the blood level of TNF-α becomes high with diseases other than septicemia such as IBD (inflammatory bowel disease), SLE (systemic lupus erythematosus), the Kawasaki disease, and the like wherein an immunity abnormality is assumed as a cause of the diseases, and further, it is known that the concentration of TNF-α in joint liquid rises in the case of RA (rheumatoid arthritis) ("Nippon Rinsho", 48, irregularly, 304-311, (1990)). Accordingly, for these diseases, a therapy to remove TNF-α from a body fluid is considered.

Japanese Unexamined Patent Publication No. 211900/1994; EP-A-0 592 989 (an application by B. Braun Melsungen Aktiengesellschaft) discloses an adsorbent for removing TNF-α from a body fluid, which comprises a porous carrier to which a polyanion polymer is covalently bonded and in the Example thereof a porous carrier is employed to which a dextran sulfate is bonded.

However, it has been proven by the inventors of the present invention that in the porous carrier to which a dextran sulfate is covalently bonded, which is described in Japanese Unexamined Patent Publication No. 211900/1994, interaction between TNF-α and the dextran sulfate is weak and that when a liquid containing TNF-α is applied to a column which is charged with the porous carrier to which the dextran sulfate is bonded, TNF-α is not adsorbed and it is only eluted from the column later. EP-A-0 321 703 is directed to an adsorbent for serum amyloid protein comprising a water-insoluble carrier and a compound immobilized thereon being an anionic functional group, a polyanionic compound having more than one anionic functional groups, aniline or an aniline derivative or a water-insoluble carrier with a group represented by the formula NR¹R² wherein R¹ and R² are as specified. EP-A-0 306 617 is directed to an autoantibody adsorbent and apparatus for removing autoantibodies comprising and using water-insoluble porous material having immobilized thereon a compound with an anionic functional group.

Also it has been similarly proven that preferable results can not be obtained when employing a sulfonated polysaccharide such as a heparin or chondroitin sulfate, instead of the dextran sulfate, a polypeptide such as polyglutamic acid or polyaspartic acid, and a nucleic acid, and further a polyacrylic acid and polyvinylsulfate.

The present invention has been accomplished in order to improve disadvantages of the conventional adsorbent comprising a porous carrier to which a polyanion polymer is bonded as mentioned above and to give an adsorbent which can adsorb and remove TNF-α selectively from a body fluid, in particular, blood, plasma or serum.

The invention relates to an adsorbent of TNF-α, wherein onto a water-insoluble carrier is immobilized a compound having a functional group represented by the formula (I): 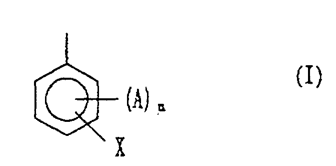 wherein X is a sulfonic acid group; A is a substituent group except for the sulfonic acid group; n is an integer of 0 to 4 and in case n is at least 2, groups A, the number of which is n, may be the same or different from each other, wherein the compound having a functional group of the formula (I) has at least two functional groups of the formula (I) in a molecule (Claim 1), the adsorbent of Claim 1, wherein the compound having at least two functional groups of the formula (I) in a molecule is polystyrenesulfonic acid (Claim 2),
the adsorbent of Claim 1 or 2, wherein the water-insoluble carrier is hydrophilic (Claim 3),
the adsorbent of Claim 3, wherein the water-insoluble carrier has a hydroxyl group (Claim 4),
the adsorbent of Claims 1 to 4, wherein the water-insoluble carrier is porous (Claim 5),
a process for adsorbing and removing TNF-α, which comprises bringing the adsorbent of Claim 1 into contact with a body fluid containing TNF-α (Claim 6),
an adsorber for adsorbing TNF-α, wherein a vessel having an inlet and an outlet for a body fluid and being equipped with a means for preventing the adsorbent from flowing to the outside of the vessel is charged with the adsorbent of Claims 1 to 5 (Claim 7),
and use of a compound having a functional group represented by the formula (I): 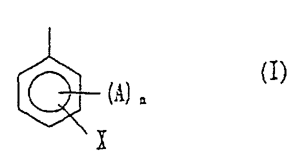 wherein X is a sulfonic acid group, A is a substituent group except for the sulfonic acid group, n is an integer of 0 to 4 and in case n is at least 2, groups A, the number of which is n, may be the same or different from each other, wherein the compound having a functional group of the formula (I) has at least two functional groups of the formula (I) in a molecule, the compound being immobilized onto a water-insoluble carrier, for the manufacture of an adsorbent for a new and inventive adsorption of TNF-α (Claim 8).

A body fluid in the present invention means blood, plasma, serum, ascites, lymph, synovial fluid, a fractionated component obtained from these fluids, or a liquid component from the other living body.

TNF-α is a simple protein having a molecular weight of 17kDa which comprises 157 amino acids and usually forms a trimer. Accordingly, the molecular weight of the trimer is approximately 51,000.

FIG. 1 is a schematic cross section of one example of the adsorber for adsorbing TNF-α of the present invention.

FIG. 2 is a graph showing the result of the examination of the relationship between the flow rate and the pressure loss ΔP by using three kinds of water-insoluble carriers.

FIG. 3 is a graph showing the change of the concentration of TNF-α and a protein in the elution liquid when serum containing TNF-α was flowed through the adsorber for adsorbing TNF-α of EXAMPLE 2, wherein the column is charged with cellulose beads CK-A3 onto which polystyrenesulfonic acid is immobilized.

FIG. 4 is a graph showing the change of the concentration of TNF-α and a protein in the elution liquid when serum containing TNF-α was flowed through the adsorber for adsorbing TNF-α of COMPARATIVE EXMAPLE 3, wherein the column is charged with CELLULOFINE GC-700m onto which sodium dextran sulfate is immobilized.

A compound used in the present invention, which is immobilized onto a water-insoluble carrier and has a functional group represented by the formula (I): 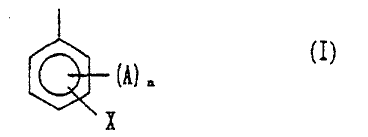 wherein X is a sulfonic acid group, A is a substituent group except for the sulfonic acid group, n is an integer of 0 to 4 and in case n is at least 2, groups A, the number of which is n, may be the same or different from each other, wherein the compound having a functional group of the formula (I) has at least two functional groups of the formula (I) in a molecule as mentioned above, is a compound which has a benzene ring to which one sulfonic acid group is bonded wherein at the remaining four positions of the benzene ring hydrogen atoms or the substituent groups except for the sulfonic acid group may be bonded. Also, at a part of the four positions of the benzene ring the substituent group except for the sulfonic acid group can be bonded. When there are at least two of the above mentioned substituent groups, they may be the same or different from each other.

The sulfonic acid group is a functional group which is charged negatively at a pH value around neutral. The group is a strong acid and is therefore particularly useful for the present invention.

The substituent group (A) which is bonded to the benzene ring of the formula (I) is not limited so long as it is a substituent group other than the sulfonic acid group, and can be an alkyl group such as a methyl group or an ethyl group or an aromatic group such as a phenyl group and can be a group which does not contain any carbon atom, such as a nitro group or a hydroxyl group.

Concrete examples of the compound having the functional group of the formula (I) are functional groups represented by the formula (II): 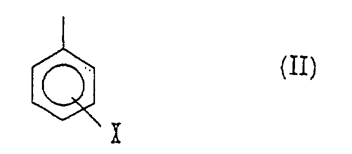 wherein X is the same as defined above, there are, for instance, sulfobenzoic acid, benzenedisulfonic acid, aminobenzenesulfonic acid (sulfanilic acid), phenolsulfonic acid, and the like, however, are not limited thereto.

Concrete examples of the compound having the functional group of the formula (I) are functional groups represented by the formula (III): 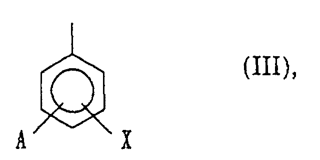 the formula (IV): 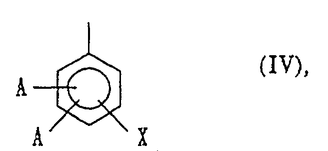 the formula (V): 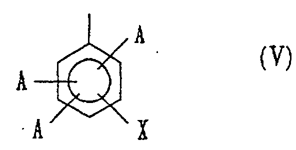 or
the formula (VI): 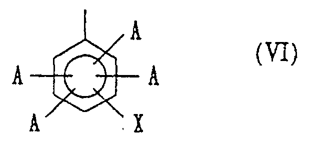 wherein X and A are the same as defined above and when at least two groups of A are bonded, they may be the same or different from each other, there are, for instance, aminohydroxybenzenesulfonic acid, chloro-methyl-nitrobenzene sulfonic acid, and the like, however, are not limited thereto.

The compound having the functional group of the formula (I) is a compound having at least two of the functional groups in a molecule. This compound has a high affinity to TNF-α and many of the functional groups can be easily introduced into an unit volume of a solid substance. Particularly, a compound having a molecular weight of at least 1000 is more preferable because it has a high affinity to TNF-α and many of the functional groups can be easily introduced into an unit volume of a solid substance.

The functional groups may be the same or different from each other.

As the concrete examples of such compound having at least two functional groups in a molecule, there are, for example, polystyrenesulfonic acid, polystyrenephosphoric acid, and the like, however, it is not limited thereto. Among them, polystyrene sulfonic acid is preferable because it has sulfonic acid group which is strong acid.

As a water-insoluble carrier used in the present invention, there are, for instance, an inorganic carrier such as glass beads or silica gel, a water-insoluble carrier comprising a synthetic polymer such as polyvinyl alcohol, a saponificated ethylene-vinyl acetate copolymer, poly(meta)acrylate such as polymethyl methacrylate, poly(meta)acrylic acid, polyacrylamide, polystyrene, polyacrylic acid-grafted polyethylene or polyacrylamide-grafted polyethylene; an organic carrier comprising a polysaccharide such as crystalline cellulose, cellulose, agarose, dextran or chitosan; and further a composite carrier obtained from a combination of the above-mentioned compounds such as an organic-organic carrier and organic-inorganic carrier, however, it is not limited thereto.

These carriers may be used alone or may be used by combining arbitrarily 2 or more kinds thereof. Among them, a hydrophilic carrier is preferable because non-specific adsorption is comparatively little and selective adsorption of TNF-α is good.

The hydrophilic carrier mentioned above means a carrier having a contact angle of at most 60 degrees with water. Such a contact angle is present with a compound constituting the carrier which is made to be in the form of a flat plate. Examples of such carriers include carriers comprising a polysaccharide such as cellulose, chitosan, agarose, dextran, or the like; a hydrophilic synthetic polymer such as poly(vinyl alcohol), a saponified ethylene-vinyl acetate copolymer, polyacrylamide, poly(acrylic acid), poly(methacrylic acid), poly(acrylic acid-grafted polyethylene), poly(acrylamide-grafted polyethylene), or the like; and glass.

Among the water-insoluble carriers mentioned above, particularly a hydrophilic carrier having a hydroxyl group is excellent in its ability for adsorption and selectivity. Among them, a porous cellulose gel is the most preferable one for the carrier used in the present invention because of such superior points as follows:
① The carrier of the porous cellulose gel is hardly destroyed or generates finely divided particles by the operation of agitation, and the like because of a relatively high mechanical strength and toughness. The carrier is neither compacted nor clogged up when a column is charged therewith and a body fluid is passed through the column at a high flow rate. Further, the pore structure hardly changes by high-pressure steam sterilization.
② The carrier is hydrophilic since the gel is constituted by cellulose. There are present many hydroxyl groups available for bonding a ligand, and the non-specific adsorption is little.
③ If the volume of porosity is enlarged, comparable adsorption volume can be obtained to a soft gel since the strength is relatively high.
④ Safety is high compared to a synthetic macromolecular gel, and the like.

The adsorbent of the present invention can adsorb TNF-α only at the outer surface, however, in order to adsorb more TNF-α, it is preferable to have a lot of pores of suitable size, that is, the adsorbent is porous. In order to adsorb efficiently TNF-α (molecular weight of the trimer: approximately 51,000), it is preferable that TNF-α can enter the pores with a sufficiently high probability whereas the penetration of other proteins occurs as little as possible.

Although a mercury porosimetry is most frequently used for measuring a pore size, in the porous water-insoluble carrier used in the invention, the mercury porosimetry cannot be applied so often since the structure of the carrier is changed on drying. Accordingly, it is suitable to employ the molecular weight of exclusion limit as a measure of the pore size of the pore.

The term "the molecular weight of exclusion limit" means the minimum molecular weight of the molecule at which the molecule cannot enter a pore (i.e. the molecule is excluded) in a gel permeation chromatography (see Hiroyuki Hatano and Toshihiko Hanai, "Experimental High Performance Liquid Chromatography", Kagaku Dojin Co., Ltd.).

Generally the molecular weight of exclusion limit for a globular protein, dextran, polyethylene glycol, or the like has been studied in detail, and in the carrier used in the invention, it is suitable to employ a value obtained using the globular protein.

Since the molecular weight of trimeric TNF-α is approximately 51,000, when a carrier having a molecular weight of exclusion limit of less than 5 × 10⁴ is used, the amount of adsorbing and removing TNF-α is low and the practicability is decreased. Accordingly, the preferable molecular weight of exclusion limit of the carrier used for TNF-α is at least 5 × 10⁴. No problems occurr as long as plasma or serum is used as a body fluid even if the molecular weight of exclusion limit is over 5 × 10⁶. However, in the case blood is used as a body fluid, there is the tendency that the degree of adhesion of platelets increases when the molecular weight of exclusion limit is over 5 × 10⁶. Accordingly, in the case the adsorbent of the invention is applied to a homocatharsis system by direct hemo-perfusion (DHP), an efficient ability of it cannot be necessarily exhibited. On the other hand, in the case the molecular weight of exclusion limit is at most 5 × 10⁶, no serious trouble is particularly caused by any usage. Thus it is desirable that the molecular weight of exclusion limit is at most 5 × 10⁶ in order to be able to use the adsorbent in many usages.

With respect to the adsorption ability per unit volume of the adsorbent, it is preferable that the adsorbent has many pores on its surface and it is preferable that the pore volume is at least 20 % and the specific surface area is at least 3 m²/g. The form of the carrier can be arbitrary such as granular, fibrous or hollow.

Further, it is preferable that there is a functional group on the carrier surface, which can be used. for immobilizing a ligand. As representative examples of such functional groups, there are, for instance, a hydroxyl group, an amino group, an aldehyde group, a carboxyl group, a thiol group, a silanol group, an amide group, an epoxy group, a halogen group, a succinylimide group, an acid anhydrous group, and the like.

As the water-insoluble carrier to be used in the invention, each of a hard carrier and a soft carrier can be used. It is important that a column is not clogged by the carrier when it is charged with the carrier and a fluid can pass therethrough in order to use the carrier as an adsorbent for extracorporeal circulation treatment. Since sufficient mechanical strength is needed to satisfy the above requirement, it is more preferable that the carrier is the hard carrier. The term "hard carrier" means, that a gel, for example, is a granulated gel as shown in REFERENCE EXAMPLE 1 mentioned below, a carrier which has a linear relationship between a pressure loss ΔP and a flow rate up to 0.3 kg/cm² of pressure loss when a cylindrical column is charged uniformly with the gel and an aqueous fluid is passed through the column.

The adsorbent of the present invention can be obtained by immobilizing the compound having a functional group of the formula (I) onto the water-insoluble carrier, and as an immobilizing method various known methods can be used without a special limit. As the representative methods, there are, for instance,
① a method wherein a compound which does not contain the group X is immobilized onto a solid substance (the water-insoluble carrier), and then the group X is introduced,
② a method wherein a compound which contains the group X is immobilized onto the solid substance, and the like.

As a method for immobilizing these compounds onto the solid substance in the method of ① and ②, there is a method by means of physical adsorption, a method by means of ionic bond, a method by means of covalent bond, or the like and any method can be used. Since it is important that the compound having the sulfonic acid group is not released upon storage of the adsorbent, the method by means of covalent bond, which is capable make a strong immobilization, is preferable.

As the representative example of the method for introducing X in the method ①, there is a method for introducing the sulfonic acid group by treating with concentrated sulfuric acid or chlorosulfonic acid.

On the other hand, when the compound having at least two functional groups of the formula (I) in a molecule is immobilized, there is, ③ a method for graft polymerizing on the solid substance except for the above mentioned methods ① and ②.

The amount of the compound having a functional group of the formula (I), which is immobilized onto the water-insoluble carrier, can be measured as the amount of the sulfonic acid group by titration. As the amount immobilized, if for example, the water-insoluble carrier is granular, it is preferable that it is at least 0.01 mmole per 1 mℓ of the sedimentation volume. If it is less than that, the amount of TNF-α which can be adsorbed extremely reduces, and the resulting adsorbent cannot be used practically.

There are various kinds . of processes for adsorbing and removing TNF-α in a body fluid by bringing a body fluid into contact with the adsorbent wherein the compound having a functional group of the formula (I) is immobilized onto the water-insoluble carrier. As representative processes, there are, for instance, a process which comprises removing a body fluid, storing it in a bag, mixing the adsorbent therewith to adsorb and remove TNF-α, and filtrating the adsorbent to obtain the body fluid from which TNF-α is removed, a process which comprises charging a vessel having an inlet and an outlet for a body fluid and being equipped with a filter through which a body, fluid can pass and the adsorbent cannot pass, at the outlet, preferably at the outlet and the inlet, with the adsorbent, and flowing the body fluid therethrough, and the like. Any processes can be used. With respect to the latter process, however, the operation thereof is simple, and TNF-α can be removed efficiently in-line from a body fluid of a patient by incorporating the latter process into the extracorporeal circulation cycle. Accordingly, the adsorbent of the invention is suitable for this process.

In the case a column is charged with the adsorbent to be used any adsorbent can be used unless it is a fine powder, and it is preferable that the distribution of the particle size is narrow as long as plasma or serum is employed as a body fluid. However, it is preferable that the adsorbent - has enough space wherein a cell contained in blood can pass freely when blood is flowed therethrough. For example, when the adsorbent is granular, a fine powder is not preferable as mentioned above, and a particle having a particle size of at least 100 µm is preferable. Further, the adsorbent wherein a particle having a too large or too small particle size is removed is preferable, and an adsorbent wherein the average of the particle size is in a range from 100 µm to 1000 µm and the distribution of the particle size is narrow, is more preferable. When the adsorbent is fibrous and hollow, it is preferable that the inner diameter is at least 5 µm. In the case the adsorbent is fibrous but not hollow, it is preferable that the diameter is at least 1 µm.

In order to avoid a non-specific adsorption of a blood corpuscle on passing blood through the adsorbent of the invention, the adsorbent may be coated with a polymer which is superior in blood compatibility, for example, a polymer of hydroxy ethyl(metha)acrylate. Such coating can be done in order to prevent the formation of fine particles of the adsorbent.

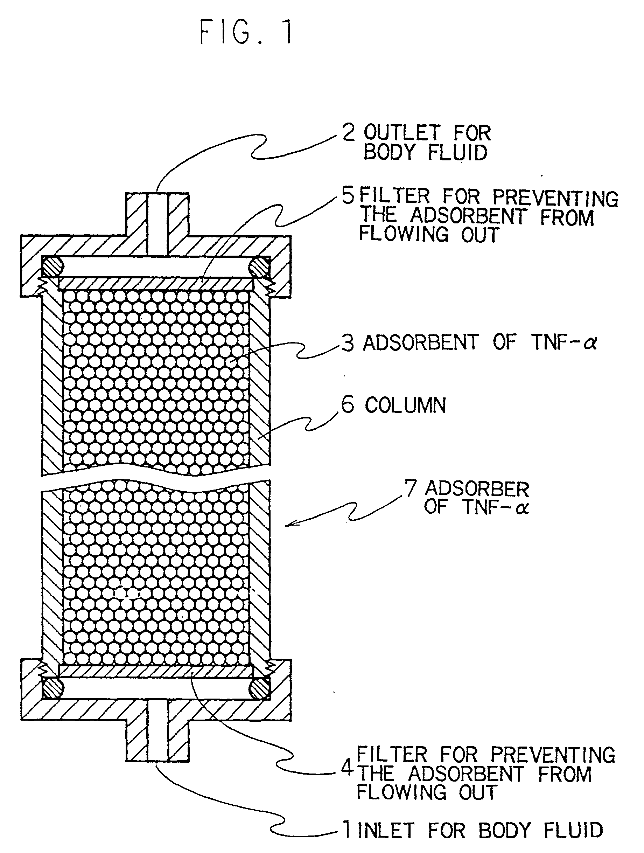

In the following, an adsorber of TNF-α, which comprises the adsorbent of the present invention, is explained by referring to FIG. 1 that is a schematic cross section of one example of the invention.

In the FIG. 1 represents an inlet for a body fluid, 2 represents an outlet for a body fluid, 3 represents an adsorbent of TNF-α of the invention, 4 and 5 represent a filter for preventing the adsorbent from flowing out, whereby the body fluid and components contained in the body fluid can pass but the adsorbent of TNF-α cannot pass, 6 represents a column and 7 represents an adsorber of TNF-α.

There is no limitation in particular with respect to the shape and material of the container of the above adsorber for TNF-α. Examples of the container include a cylindrical column with a capacity of 150 to 400 mℓ and a diameter of 4 to 10 cm.

The present invention is explained in detail by means of the following EXAMPLES, however, the invention is not limited to the following EXAMPLES.

### REFERENCE EXAMPLE 1

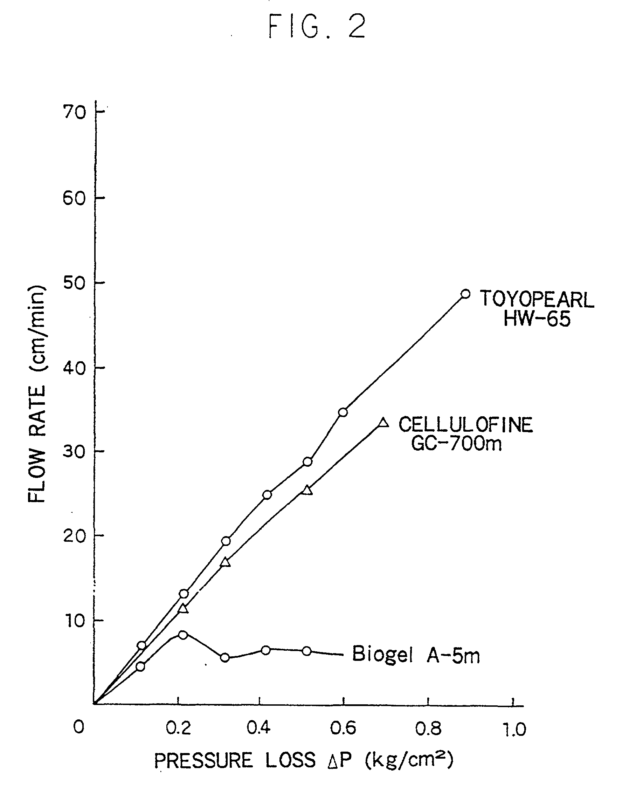

Each of cylindrical columns of glass (9 mm in inner diameter, 150 mm in length of the column) equipped with filters having a pore size of 15 µm on both ends, was charged uniformly with an agarose gel (Biogel A-5m made by BIO-RAD, 50 to 100 meshes in particle size), a vinyl polymer gel (TOYOPEARL HW-65 made by TOSOH Corporation, 50 to 100 µm in particle size) or a cellulose gel (CELLULOFINE GC-700m made by CHISSO CORPORATION, 45 to 105 µm in particle size). And then, each of the relationships between the flow rate and pressure loss ΔP was determined by passing water through each of the columns with a peristaltic pump. The results are shown in FIG. 2.

As shown in FIG. 2, it is realized that each flow rate in the cases of TOYOPEARL HW-65 and CELLULOFINE GC-700m increases almost in proportion as the pressure increases. On the other hand, it is realized that Biogel A-5m causes a compactness and the flow rate does not increase when the pressure is increased.

In the present invention, as in the former case, the gel in which the relationship between the pressure loss ΔP and the flow rate is in linear relationship up to 0.3 kg/cm² is defined as a hard gel.

### EXAMPLE 1

Preparation of the adsorbent: In a reaction vessel, 100 mℓ of cellulose beads CK-A3 (made by CHISSO CORPORATION, 5 × 10⁷ in molecular weight of exclusion limit for a globular protein), 100 mℓ of water, 50 mℓ of 2N sodium hydroxide and 20 mℓ of epichlorohydrin were mixed and reacted for 2 hours at 40°C to obtain epoxidated cellulose beads CK-A3. In a reaction vessel, 100 mℓ of the resulting epoxidated cellulose beads CK-A3, 100 mℓ of water and 10 mℓ of 28 % ammonia water were mixed and reacted over night at room temperature to obtain aminated cellulose beads CK-A3.

On the other hand, 10 g of poly(sodium styrenesulfonate) (7 × 10⁴ in average molecular weight), 1 mℓ of thionyl chloride and 250 mℓ of toluene were mixed in a reaction vessel and reacted for 8 hours at room temperature to give partially chlorinated poly(sodium styrenesulfonate).

In a reaction vessel, 10 g of the resulting chlorinated poly(sodium styrenesulfonate), 100 mℓ of the aminated cellulose beads CK-A3 and 100 mℓ of water were admixed and reacted over night at room temperature to obtain poly(sodium styrenesulfonate)-immobilized cellulose beads CK-A3. It was confirmed that poly(sodium styrenesulfonate) containing approximately 30 mmoles of the sulfonic acid group per 1 mℓ of sedimention volume, was immobilized by means of a titration method.

Evaluation of the adsorbent: The resulting poly(sodium styrenesulfonate)-immobilized cellulose beads CK-A3 were washed with physiological saline. These beads (0.5 mℓ) were put in a test tube, and excess physiological saline was removed. Thereto, 3 mℓ of human serum containing approximately 20 ng/mℓ of TNF-α were added and shaked for 2 hours at 37°C. The concentration of TNF-α in the supernatant was measured by means of an ELISA method. The results of the analysis are shown in TABLE 1.

### COMPARATIVE EXAMPLE 1

Cellulose beads CK-A3 were equilibrated with physiological saline. These beads (0.5 mℓ) were put in a test tube, and excess physiological saline was removed. Thereto, 3 mℓ of human serum containing about 20 ng/mℓ of TNF-α were added and shaken for 2 hours at 37°C. The concentration of TNF-α in the supernatant was measured by means of an ELISA method. The results are shown in TABLE 1.

### COMPARATIVE EXAMPLE 2

Preparation of the adsorbent: In a reaction vessel, 100 mℓ of cellulose beads CK-A3, 100 mℓ of water, 50 mℓ of 2N sodium hydroxide and 20 mℓ of epichlorohydrin were mixed and reacted for 2 hours at 40°C to obtain epoxidated cellulose beads CK-A3. 6 g of sodium dextran sulfate and 100 mℓ of water (the concentration of sodium dextran sulfate was approximately 2.5 %) were added to 100 mℓ of the resulting epoxidated cellulose beads CK-A3, and adjusted to pH 11 and shaken for 16 hours at 45°C. And then, the resulting gel was filtered off and washed with water to obtain sodium dextran sulfate-immobilized cellulose beads CK-A3. It was confirmed that sodium dextran sulfate containing approximately 30 mmoles of the sulfate group per 1 mℓ of sedimentation volume was immobilized by means of a titration method.

Evaluation of the adsorbent: The resulting sodium dextran sulfate-immobilized cellulose beads CK-A3 were washed with physiological saline. These beads (0.5 mℓ) were put in a test tube, and excess physiological saline was removed. Thereto, 3 mℓ of human serum containing approximately 20 ng/mℓ of TNF-α were added and shaken for 2 hours at 37°C. The concentration of TNF-α in the supernatant was measured by means of an ELISA method. The results of the analysis are shown in TABLE 1.

**TABLE 1**

| | Concentration of TNF-α in the supernatant |
|---|---|
| EX. 1 | 16 ng/mℓ |
| COM. EX. 1 | 21 ng/mℓ |
| COM. EX. 2 | 21 ng/mℓ |

It is easily found that the concentration of TNF-α in EXAMPLE 1 decreases compared with that in COMPARATIVE EXAMPLE 1 and that TNF-α in a body fluid can be efficiently adsorbed and removed by using the adsorbent of the present invention. In contrast, it is found from COMPARATIVE EXAMPLE 2, that TNF-α cannot be adsorbed by an adsorbent wherein a compound merely having group X, sodium dextran sulfate, is immobilized and that X which is bonded to a benzene ring is necessary.

### EXAMPLE 2

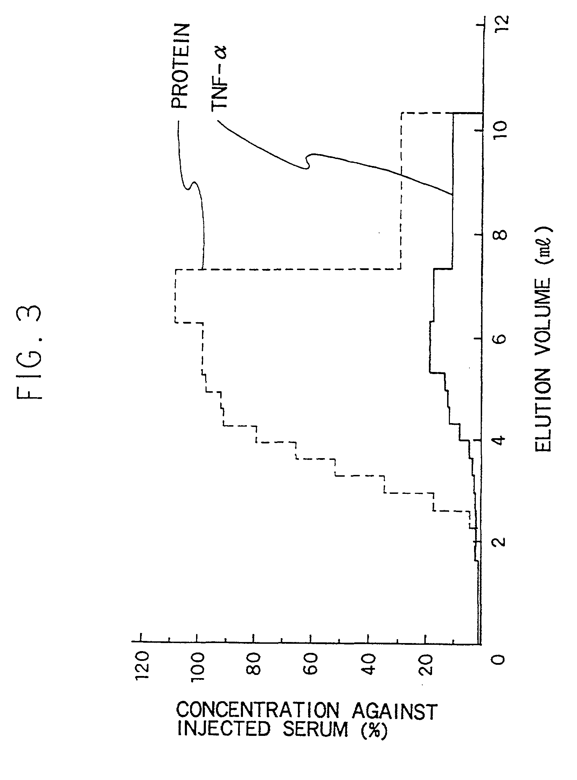

A column (made by BIO-RAD, 10 mm in inner diameter, 100 mm in length of the column) was charged with 2 mℓ of poly(sodium styrenesulfonate)-immobilized cellulose beads CK-A3 obtained in EXAMPLE 1 and washed with physiological saline. And then, therethrough 5.3 mℓ of human serum containing approximately 20 ng/mℓ of TNF-α were passed at a flow rate of 0.33 mℓ/min and subsequently 5 mℓ of physiological saline were passed at the same flow rate. During such procedure, the resulting eluent from the outlet of the column was collected, in which firstly 0.33 mℓ of each fraction was collected 16 times, subsequently 1 mℓ of each fraction was collected twice and finally 3 mℓ of a fraction was collected once. The concentration of TNF-α in each fraction was measured by means of an ELISA method and the concentration of protein was also measured by employing BCA Protein Assay Reagent (made by PIERCE). The results are shown in FIG. 3.

The concentration of TNF-α or protein in each fraction in FIG. 3 is represented as a percentage whereby the concentration of TNF-α or protein in the injected serum is 100.

### COMPARATIVE EXAMPLE 3

Preparation of the adsorbent: In a reaction vessel, 100 mℓ of CELLULOFINE GC-700m (made by CHISSO CORPORATION, 4 × 10⁵ in molecular weight of exclusion limit for a globular protein), 100 mℓ of water, 50 mℓ of 2N sodium hydroxide and 20 mℓ of epichlorohydrin were mixed and reacted for 2 hours at 40°C to obtain epoxidated CELLULOFINE GC-700m. 6 g of sodium dextran sulfate and 100 mℓ of water (the concentration of sodium dextran sulfate was approximately 2.5 %) were added to 100 mℓ of the resulting epoxidated CELLULOFINE GC-700m and adjusted to pH 11 and shaken for 16 hours at 45°C. And then, the resulting gel was filtrated off and washed with water to obtain sodium dextran sulfate-immobilized CELLULOFINE GC-700m. It was confirmed that sodium dextran sulfate containing approximately 30 mmoles of the sulfate group per 1 mℓ of sedimentation volume was immobilized by means of a titration method.

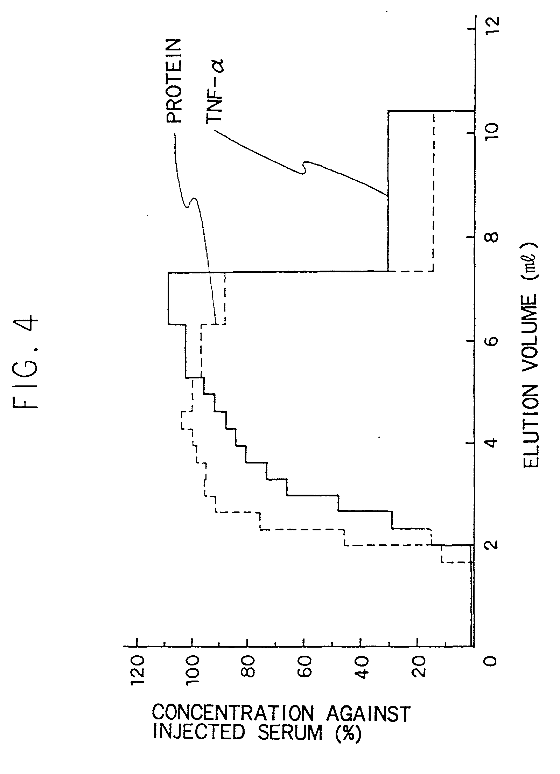

Evaluation of the adsorbent: A column (made by BIO-RAD) was charged, in the same way as in EXAMPLE 2, with 2 mℓ of the resulting sodium dextran sulfate-immobilized CELLULOFINE GC-700m and washed with physiological saline. And then, therethrough were passed 5.3 mℓ of human serum containing approximately 20 ng/mℓ of TNF-α at a flow rate of 0.33 mℓ/min and subsequently 5 mℓ of physiological saline were passed at the same flow rate. During such procedure, the resulting eluent from the outlet of the column was collected in the same way as in EXAMPLE 2 and the concentration of TNF-α in each fraction was measured by means of an ELISA method and the concentration of protein was also measured. The results are shown in FIG. 4.

The concentration of TNF-α or protein in each fraction in FIG. 4 is represented as a percentage whereby the concentration of TNF-α or protein in the injected serum is 100.

It seems that TNF-α is apparently adsorbed, because the first elution of TNF-α is slow compared with that of protein in COMPARATIVE EXAMPLE 3 as shown FIG. 4. However, it is found that with passing serum the concentration of TNF-α in the eluent exceeds that of the protein in the serum and that when subsequently passing physiological saline more TNF-α is eluted than protein. It is realized eventually that almost all amount of TNF-α is eluted when comparing the protein with TNF-α in the area of the graph.

Namely, it is found that TNF-α is practically not adsorbed in COMPARATIVE EXAMPLE 3 and the adsorbent thereof cannot be used actually.

On the other hand, it is found that the concentration of TNF-α in the eluent hardly increases in FIG. 3 and that TNF-α can surely be adsorbed and removed.

Using the adsorbent of the present invention for adsorbing and removing TNF-α in a body fluid, TNF-α can be efficiently adsorbed and removed compared with a conventional adsorbent.

Also, using the adsorber of the invention which is charged with the adsorbent of the invention for adsorbing and removing TNF-α in a body fluid, TNF-α can be continuously adsorbed and removed.

## Claims

1. An adsorbent of tumor necrosis factor-α, wherein onto a water-insoluble carrier is immobilized a compound having a functional group represented by the formula (I): 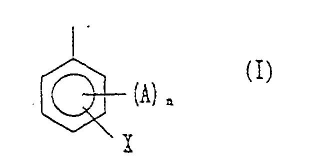 wherein X is a sulfonic acid group, A is a substituent group except for the sulfonic acid group, n is an integer of 0 to 4 and in case n is at least 2, groups A, the number of which is n, may be the same or different from each other, wherein the compound having a functional group of the formula (I) has at least two functional groups of the formula (I) in a molecule.

2. The adsorbent of Claim 1, wherein the compound having at least two functional groups of the formula (I) in a molecule is polystyrenesulfonic acid.

3. The adsorbent of Claim 1 or 2, wherein the water-insoluble carrier is hydrophilic.

4. The adsorbent of Claim 3, wherein the water-insoluble carrier has a hydroxyl group.

5. The adsorbent of any one of Claims 1 to 4, wherein the water-insoluble carrier is porous.

6. A process for adsorbing and removing tumor necrosis factor- α, which comprises bringing the adsorbent of Claim 1 into contact with a body fluid containing tumor necrosis factor-α.

7. An adsorber for adsorbing tumor necrosis factor-α , wherein a vessel having an inlet and an outlet for a body fluid and being equipped with a means for preventing the adsorbent from flowing to the outside of the vessel, is charged with the adsorbent of any one of Claims 1 to 5.

8. Use of a compound having a functional group represented by the formula (I): 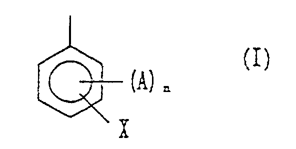 wherein X is a sulfonic acid group, A is a substituent group except for the sulfonic acid group, n is an integer of 0 to 4 and in case n is at least 2, groups A, the number of which is n, may be the same or different from each other, wherein the compound having a functional group of the formula (I) has at least two functional groups of the formula (I) in a molecule, the compound being immobilized onto a water-insoluble carrier, for the manufacture of an adsorbent for a new and inventive adsorption of tumor necrosis factor- α.

## Patentansprüche

1. Adsorbens für Tumornekrosefaktor-α, wobei auf einem wasserunlöslichen Träger eine Verbindung mit einer funktionellen Gruppe der Formel (I): 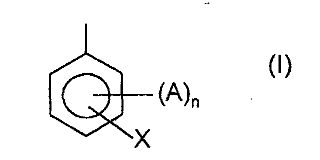 immobilisiert ist, X eine Sulfonsäuregruppe ist, A eine Substituentengruppe ist, ausgenommen für die Sulfonsäuregruppe, n eine ganze Zahl von 0 bis 4 ist und im Fall, daß n mindestens 2 ist, die Gruppen A, deren Anzahl n ist, gleich sind oder unterschiedlich voneinander sind, wobei die Verbindung mit einer funktionellen Gruppe der Formel (I) mindestens zwei funktionelle Gruppen der Formel (I) in einem Molekül hat.

2. Adsorbens nach Anspruch 1, wobei die Verbindung mit mindestens zwei funktionellen Gruppen der Formel (I) in einem Molekül Polystyrolsulfonsäure ist.

3. Adsorbens nach einem der Ansprüche 1 oder 2, wobei der wasserunlösliche Träger hydrophil ist.

4. Adsorbens nach Anspruch 3, wobei der wasserunlösliche Träger eine Hydroxylgruppe hat.

5. Adsorbens nach einem der Ansprüche 1 bis 4, wobei der wasserunlösliche Träger porös ist.

6. Verfahren zur Adsorption und Entfernung von Tumornekrosefaktor-α, umfassend das Inkontaktbringen des Adsorbens nach Anspruch 1 mit einer Körperflüssigkeit, die den Tumornekrosefaktor-α enthält.

7. Adsorber zur Adsorption von Tumornekrosefaktor-α, wobei ein Gefäß mit einem Einlaß und einem Auslaß für eine Körperflüssigkeit, ausgestattet mit einer Vorrichtung, die verhindert, daß das Adsorbens aus dem Gefäß fließt, mit dem Adsorbens nach einem der Ansprüche 1 bis 5 beladen ist.

8. Verwendung einer Verbindung mit einer funktionellen Gruppe der Formel (I): 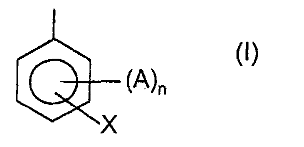 wobei X eine Sulfonsäuregruppe ist, A eine Substituentengruppe ist, ausgenommen für die Sulfonsäuregruppe, n eine ganze Zahl von 0 bis 4 ist und im Fall, daß n mindestens 2 ist, die Gruppen A, deren Anzahl n ist, gleich sind oder sich voneinander unterscheiden, wobei die Verbindung mit einer funktionellen Gruppe der Formel (I) mindestens zwei funktionelle Gruppen der Formel (I) in einem Molekül hat, wobei die Verbindung auf einem wasserunlöslichen Träger immobilisiert ist, für die Herstellung eines Adsorbens für eine neue und erfinderische Adsorption von Tumornekrosefaktor-α.

## Revendications

1. Adsorbant du facteur α de nécrose tumorale dans lequel, sur un support insoluble dans l'eau, est immobilisé un composé ayant un groupe fonctionnel représenté par la formule (I) : 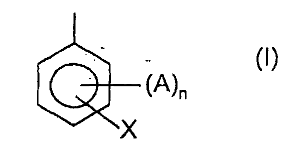 dans laquelle X est un groupe acide sulfonique, A est un groupe de substitution excepté pour le groupe acide sulfonique, n est un nombre entier de 0 à 4 et dans le cas où n vaut au moins 2, les groupes A, dont le nombre est n, peuvent être identiques ou différents les uns des autres, le composé ayant un groupe fonctionnel de formule (I) ayant au moins deux groupes fonctionnels de formule (I) dans une molécule.

2. Adsorbant selon la revendication 1, dans lequel le composé ayant au moins deux groupes fonctionnels de formule (I) dans une molécule est l'acide polystyrène-sulfonique.

3. Adsorbant selon la revendication 1 ou 2, dans lequel le support insoluble dans l'eau est hydrophile.

4. Adsorbant selon la revendication 3, dans lequel le support insoluble dans l'eau contient un groupe hydroxyle.

5. Adsorbant selon l'une quelconque des revendications 1 à 4, dans lequel le support insoluble dans l'eau est poreux.

6. Procédé pour adsorber et éliminer le facteur α de nécrose tumorale, qui comprend l'étape consistant à amener l'adsorbant selon la revendication 1 en contact avec un fluide corporel contenant le facteur α de nécrose tumorale.

7. Appareil d'adsorption pour adsorber le facteur α de nécrose tumorale, dans lequel un réacteur présentant un orifice d'entrée et un orifice de sortie pour un fluide corporel et équipé d'un moyen pour empêcher l'adsorbant de déborder du réacteur, est chargé avec l'adsorbant selon l'une quelconque des revendications 1 à 5.

8. Utilisation d'un composé ayant un groupe fonctionnel représenté par la formule (I) : 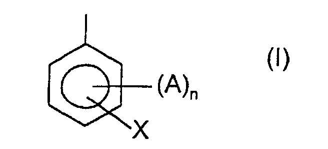 dans laquelle X est un groupe acide sulfonique, A est un groupe de substitution excepté pour le groupe acide sulfonique, n est un nombre entier de 0 à 4 et dans le cas où n vaut au moins 2, les groupes A, dont le nombre est n, peuvent être identiques ou différents les uns des autres, le composé ayant un groupe fonctionnel de formule (I) ayant au moins deux groupes fonctionnels de formule (I) dans une molécule et étant immobilisé sur un support insoluble dans l'eau, pour la fabrication d'un adsorbant pour un nouveau procédé d'adsorption du facteur α de nécrose tumorale selon la présente invention.
